# FASCICULE DE BREVET EUROPEEN

(11) **EP 2 706 965 B1**
(45) Date de publication et mention de la délivrance du brevet: **01.10.2014**
(21) Numéro de dépôt: 12719973.5
(22) Date de dépôt: 09.05.2012
(51) Int. Cl.: A61F 5/56, A61C 7/06, A61C 7/08

(54) **DISPOSITIF DE TRAITEMENT ET/OU DE SOULAGEMENT DES TROUBLES RESPIRATOIRES OBSTRUCTIFS DU SOMMEIL**
VORRICHTUNG ZUR BEHANDLUNG UND/ODER LINDERUNG VON RESPIRATORISCHEN OBSTRUKTIVEN SCHLAFSTÖRUNGEN
DEVICE FOR THE TREATMENT AND/OR ALLEVIATION OF OBSTRUCTIVE RESPIRATORY SLEEP DISORDERS

(30) Priorité: 13.05.2011 FR 1154149
(43) Date de publication de la demande: 19.03.2014
(73) Titulaire: Bonnaure, Pierre, 35700 Rennes (FR)
(72) Inventeur: Bonnaure, Pierre, 35700 Rennes (FR)
(74) Mandataire: Maillet, Alain
(86) Numéro de dépôt international: PCT/EP2012/058542
(87) Numéro de publication internationale: WO 2012/156247

(56) Documents cités:
- WO-A1-01/52928
- US-A- 4 650 249
- US-A- 5 971 485

## Description

La présente invention concerne un dispositif de traitement et/ou de soulagement des troubles respiratoires obstructifs du sommeil.

Les troubles respiratoires obstructifs du sommeil, tels que les Syndromes d'Apnées Hypopnées Obstructives du Sommeil (SAHOS), sont définis par la survenue durant le sommeil d'obstruction complète ou partielle des voies aériennes supérieures. Ces phénomènes sont responsables d'interruptions, c'est à dire d'apnées, ou de baisses, c'est à dire d'hypopnées, de la ventilation, ayant pour conséquences visibles des ronflements, des altérations du sommeil ou des somnolences diurnes.

Chez l'enfant, les troubles respiratoires obstructifs du sommeil sont également courants. On estime en effet que 2 à 10 % de la population pédiatrique souffrent de tels troubles. Ils ont pour conséquences différents problèmes, tels que des problèmes de croissance, des problèmes d'hyperactivité, des problèmes de concentration ou encore des troubles du développement des maxillaires et du visage.

L'origine de ces phénomènes réside en un collapsus du pharynx, c'est à dire une obturation des voies aériennes supérieures à l'inspiration. Le collapsus peut avoir différentes origines et peut être lié, par exemple, à une luette trop volumineuse, un voile du palais trop épais, ou une mauvaise position de la base de la langue. Une autre cause fréquente de ces phénomènes est liée à un trouble de la croissance antéro-postérieure de la mandibule. En effet, la position relative des maxillaires inférieur et supérieur l'un par rapport à l'autre joue ainsi un rôle considérable sur le diamètre pharyngé.

La médecine dispose, depuis 1981, d'un traitement efficace mais à la fois lourd et coûteux : la ventilation par pression positive continue (VPPC). Durant la nuit, le patient doit porter un masque lui insufflant de l'air par le nez au travers d'un tuyau. En plus d'être coûteux, certains patients ne supportent pas le bruit occasionné par la machine.

Il est également possible d'avoir recours à la chirurgie, par exemple, pour couper le voile du palais lorsqu'il est trop long ou pour avancer le menton. L'intervention chirurgicale peut néanmoins être perçue comme relativement lourde pour le patient et comporte les risques habituels liés à toute intervention chirurgicale.

Une autre solution moins onéreuse consiste en l'utilisation d'une orthèse d'avancée mandibulaire. Le principe consiste à propulser en avant et de quelques millimètres, la mandibule par rapport à sa position d'origine par rapport au maxillaire.

Cet avancement permet le dégagement du pharynx et permet ainsi une ventilation correcte du sujet.

Par ailleurs, la prise en charge d'un problème de croissance de la mandibule chez l'enfant via le port momentané d'une orthèse d'avancée mandibulaire permet d'éviter les troubles obstructifs du sommeil à l'âge adulte. Chez l'adulte, où aucun traitement autre que chirurgical ne peut s'avérer efficace, il s'agira plutôt de soulager les patients durant leur sommeil.

Chez l'enfant encore, une étude randomisée réalisée chez 14 enfants ayant porté une telle orthèse a montré une diminution significative des ronflements, de la fatigue diurne et de l'index de désaturations, comparativement aux données de pré-traitement.

L'orthèse d'avancement mandibulaire la plus connue est sans doute la bielle de Herbst qui a vu le jour dans les années 80. Cette orthèse est principalement constituée de deux gouttières rigides prévues pour s'ancrer sur les rangées de dents du haut et du bas, respectivement, et d'une articulation composée de bielles disposées respectivement de chaque côté des arcades dentaires supérieure et inférieure de façon à relier la rangée de dents du haut et la rangée de dents du bas. De nombreuses orthèses ont été depuis dérivées de la bielle de Herbst. Par exemple, on utilise aujourd'hui une orthèse plus physiologique dont l'articulation est constituée d'une bielle et d'un triangle. Le point commun entre ces orthèses connues de l'état de la technique est que les forces exercées sur la mandibule sont intra-orales et dirigées uniquement dans un sens horaire. Une force exercée dans un sens horaire au sens de l'invention est une force orientée vers l'avant et le bas du visage vu de son profil droit. Le fait d'exercer les forces depuis un point intra-oral, limite le choix de celles-ci. Or, il peut être souhaitable d'exercer des forces dans un sens antihoraire ou de pouvoir varier ces forces chez un même patient au cours du traitement. De même, chez l'enfant, il serait souhaitable de disposer d'un dispositif permettant à la fois une expansion et une avancée du maxillaire supérieur.

Il est, par ailleurs, connu que la position de décubitus ventral est indiquée chez les patients souffrant de troubles respiratoires obstructifs du sommeil afin d'améliorer le débit d'air au cours de la ventilation. Cette position permet en effet d'ouvrir le carrefour aéro-digestif en avançant la langue.

Le but de l'invention est de proposer un dispositif de traitement et/ou de soulagement des troubles respiratoires obstructifs du sommeil :
- qui permet d'appliquer des forces sur le maxillaire supérieur et/ou sur la mandibule dans n'importe quelle direction prédéterminée, dans un sens antihoraire ou horaire,
- qui permet d'éliminer le phénomène de collapsus des voies aériennes supérieures chez l'adulte ou chez l'enfant, qui maintient une ventilation correcte du sujet durant la nuit en le positionnant de manière optimum, et qui permet d'activer et/ ou de réorienter la croissance des maxillaires et/ou de la mandibule chez l'enfant.
- qui soit utilisable indifféremment chez l'adulte comme chez l'enfant,
- qui soit adaptable à chaque morphologie du patient.

A cet effet, l'invention concerne un dispositif de traitement et/ou de soulagement des troubles respiratoires obstructifs du sommeil d'un patient qui se caractérise en ce qu'il comprend :
- un support pour le corps d'un patient dont le châssis comprend un appui pour la tête du patient, un siège et un support pour la cage thoracique lesquels sont montés sur le châssis de manière à ce que lorsque le patient prend place et s'assoit sur ledit support et positionne sa tête contre l'appui pour la tête, son buste soit maintenu incliné vers l'avant selon un angle α, à la verticale au sol, inférieur à 90°,
- un appareil d'avancée mandibulaire composé d'un moyen extra-oral d'ancrage à la mandibule et/ou d'un moyen intra-oral d'ancrage à la mandibule,
   et/ou
- un appareil d'avancée du maxillaire supérieur composé d'un moyen intra-oral d'ancrage au maxillaire supérieur,
- des moyens de fixation, sur ledit appui pour la tête, d'un ou plusieurs moyens prévus pour exercer une ou plusieurs forces prédéterminées sur la mandibule et/ou le maxillaire supérieur par l'intermédiaire de l'appareil d'avancée mandibulaire et/ou de l'appareil d'avancée du maxillaire supérieur.

Au sens de l'invention, le traitement et/ou le soulagement des troubles respiratoires obstructifs du sommeil incluent la prévention et/ou le traitement des troubles de développement des maxillaires et de la mandibule chez l'enfant et, en particulier, l'activation et la réorientation de la croissance du maxillaire supérieur et de la mandibule.

Grâce au dispositif selon l'invention, les forces à appliquer sur la mandibule et/ou le maxillaire supérieur sont générées depuis des points extra-oraux présents sur l'appui pour la tête du support pour le corps et donc depuis l'environnement externe du visage du patient. L'appui pour la tête offre un grand choix quant aux positions des moyens de fixation des moyens exerçant des forces. La détermination des forces n'est ainsi pas limitée par l'espace buccal restreint du patient. Les forces peuvent être exercées selon n'importe quelle direction prédéterminée. Les forces sont transmises à la mandibule et/ou le maxillaire supérieur par l'intermédiaire des appareils d'avancée mandibulaire et/ou d'avancée du maxillaire supérieur. Le dispositif selon l'invention permet également de combiner les avantages d'un support permettant de positionner le patient dans une position dans laquelle ses voix aériennes supérieures sont les plus désencombrées possibles et ceux des appareils de repositionnement des maxillaires.

Le dispositif selon l'invention peut comprendre uniquement un appareil d'avancée mandibulaire, uniquement un appareil d'avancée du maxillaire, ou les deux à la fois, selon le patient à traiter.

Préférentiellement, lorsqu'il comprend un appareil d'avancée mandibulaire, celui-ci est composé d'un moyen intra-oral d'ancrage à la mandibule et d'un appui extra-oral sous mandibulaire, reliés entre eux par un lien extra-oral comportant préférentiellement une charnière. Dans ce cas, il s'agit généralement d'un appareil amovible, l'appui extra-oral sous mandibulaire renforçant le maintien de l'appareil en place. La présence d'une charnière a dans ce cas pour but de faciliter la mise en place et le retrait de l'appareil.

Dans une variante de l'invention, le moyen d'ancrage à la mandibule est composé d'un moyen extra-oral d'ancrage à la mandibule, préférentiellement composé d'au moins un implant transcutané. Ce mode de réalisation est adapté au traitement des nouveau-nés, par exemple ceux souffrant d'un syndrome de Pierre Robin.

Selon un mode de réalisation de l'invention, l'appui pour la tête est composé d'un cadre comportant deux premières branches latérales, parallèles entre elles, et une surface d'appui pour le front du patient s'étendant entre lesdites premières branches latérales, lesdites premières branches latérales encadrant la tête du patient lorsque celui-ci prend place sur ledit support et positionne son front contre la surface d'appui. Le cadre forme dans ce cas généralement un U inversé.

Selon un mode de réalisation préféré de l'invention, les branches latérales du cadre sont doublées, respectivement, de deux secondes branches latérales parallèles entre elles, ces dernières se trouvant dans un plan parallèle au plan contenant les premières branches latérales.

Les premières branches latérales se trouvent dans un premier plan et les secondes branches latérales se trouvent dans un plan parallèle au premier. Au sens de l'invention, et dans ce qui suit, on entendra par « premières branches latérales », les branches latérales de l'appui pour la tête qui se trouveront dans le plan destiné à être le plus proche du visage du patient et « secondes branches latérales » les branches latérales de l'appui pour la tête qui se trouveront dans le plan destiné à être le plus éloigné du visage du patient, par rapport au plan des « premières branches ».

Ainsi selon ce mode de réalisation de l'invention, les secondes branches latérales se trouvent dans un plan postérieur au plan des premières branches par rapport au visage du patient lorsque celui-ci prend place sur ledit support et positionne son front contre la surface d'appui.

Selon un mode de réalisation de l'invention, au moins un arceau relie les premières branches latérales entre elles et/ou au moins un arceau relie les secondes branches latérales entre elles, de manière à ce que le ou les arceaux se trouvent face au visage dudit patient lorsque celui-ci prend place sur ledit support et positionne son front contre la surface d'appui.

Les branches latérales étant disposées dans l'environnement des tempes ou du profile du patient lors de l'utilisation du dispositif, les arceaux qui s'étendent d'une branche latérale à l'autre entourent de ce fait la quasi totalité du visage du patient.

Avantageusement, les moyens de fixation des moyens prévus pour exercer une ou plusieurs forces prédéterminées sont disposés sur au moins un des arceaux. Préférentiellement, lorsque le dispositif comprend quatre branches latérales, les moyens de fixations sont disposés sur le ou les arceaux qui s'étendent entre les branches latérales dont le plan est le plus éloigné du visage du patient.

Selon un mode de réalisation de l'invention, les moyens de fixation des moyens prévus pour exercer des forces prédéterminées sont mobiles le long des arceaux, des moyens de réglage de la position desdits moyens de fixation sur ledit ou lesdits arceaux étant alors prévus.

Avantageusement encore, les arceaux sont montés mobiles le long des branches latérales, un moyen de réglage de la position desdits arceaux entre les branches latérales étant alors prévu.

Lorsque le patient se positionne sur le dispositif, le front appuyé contre l'appui prévu à cet effet, les arceaux font face à son visage. Le nombre et la position des moyens de fixation pouvant varier le long des arceaux, ainsi que le nombre d'arceaux ou leur position entre les branches latérales, il existe un grand nombre de possibilités pour fixer à l'appui pour la tête les moyens prévus pour exercer les forces sur la mandibule et/ou le maxillaire supérieur. De ce fait, il est possible d'appliquer des forces antéro-postérieures, verticales, transversales ou des combinaisons de telles forces.

Selon une variante de l'invention, les branches latérales comprennent des points d'ancrage fixes sur lesquels ledit ou desdits arceaux sont susceptibles d'être accrochés. De même, les moyens de fixations peuvent être fixes le long des arceaux et régulièrement répartis sur ceux-ci.

Selon un mode de réalisation de l'invention, les premières et/ou secondes branches latérales de l'appui pour la tête sont conformées pour que, lorsque le patient prend place sur le support et place son front contre l'appui pour le front, une partie au moins des branches latérales encadre la mandibule et /ou le maxillaire supérieur. Par exemple, les branches peuvent comporter un coude.

Selon un mode de réalisation de l'invention, les premières et/ou secondes branches latérales de l'appui pour la tête comportent chacune au moins un moyen de fixation des moyens prévus pour exercer une ou plusieurs forces sur la mandibule. Dans ce mode de réalisation, les moyens de fixation permettront d'appliquer des forces latéralement sur la mandibule et/ou sur le maxillaire supérieur.

Selon un mode de réalisation de l'invention, l'appareil d'avancée du maxillaire supérieur comprend au moins une branche extra-orale, préférentiellement amovible, se projetant depuis le moyen intra-oral d'ancrage au maxillaire supérieur en avant du visage du patient lorsque celui-ci porte ledit appareil, à l'extrémité de laquelle sont prévus un ou plusieurs moyens d'ancrage des moyens prévus pour exercer une ou plusieurs forces prédéterminées sur le maxillaire supérieur.

La branche extra-orale est préférentiellement une tige rigide qui se prolonge hors de la cavité buccale du patient dans un plan horizontal médian de l'appareil d'avancée du maxillaire supérieur. Cette tige présente à son extrémité un moyen permettant l'ancrage d'un moyen prévu pour exercer une force sur la maxillaire supérieur lui même fixé à un moyen de fixation présent sur un arceau.

Dans une variante de l'invention, l'appareil d'avancée du maxillaire supérieur comprend une branche extra-orale qui se prolonge depuis le moyen d'ancrage au maxillaire supérieur et dont l'extrémité forme une croix munie de moyens d'ancrage des moyens prévus pour exercer une ou plusieurs forces sur le maxillaire supérieur. Ce mode de réalisation permet d'ancrer plusieurs moyens prévus pour exercer des forces.

Dans une autre variante de l'invention, l'appareil d'avancée du maxillaire supérieur comporte deux branches extra-orales formant chacune un arc se projetant depuis le moyen d'ancrage au maxillaire supérieur hors de la cavité buccale du patient, lorsque celui-ci porte ledit appareil, en direction de la partie postérieure de la tête du patient, les branches comportant chacune au moins un moyen d'ancrage des moyens prévus pour exercer une ou plusieurs forces sur le maxillaire supérieur. Dans ce mode de réalisation, les branches extra-orales suivent chacune un profil du visage du patient. Généralement, dans ce mode de réalisation, les moyens prévus pour exercer des forces sur le maxillaire supérieur seront fixés aux parties des branches latérales qui encadrent le maxillaire supérieur. Dans ce cas, il sera possible d'appliquer des forces latéralement au maxillaire supérieur.

Les moyens prévus pour exercer une ou plusieurs forces prédéterminées sur le maxillaire supérieur sont préférentiellement des élastiques orthodontiques. Les élastiques couramment utilisés en orthodontie conviennent parfaitement. En fonction de la force à appliquer, l'homme du métier sait déterminer le diamètre ainsi que l'épaisseur de l'élastique à utiliser. Avantageusement, les moyens prévus pour exercer des forces sur le maxillaire supérieur comportent des rupteurs de forces.

Selon un mode de réalisation de l'invention, l'appareil d'avancée mandibulaire comporte au moins une branche extra-orale, se projetant depuis le moyen intra-oral d'ancrage à la mandibule, ou depuis le lien extra-oral, en avant du visage du patient lorsque celui-ci porte ledit appareil, à l'extrémité de laquelle sont prévus un ou plusieurs moyens d'ancrage des moyens prévus pour exercer une ou plusieurs forces prédéterminées sur la mandibule.

La branche extra-orale est encore dans ce cas préférentiellement une tige rigide qui se prolonge hors de la cavité buccale du patient dans un plan horizontal médian de l'appareil d'avancée mandibulaire. Cette tige présente à son extrémité un moyen permettant l'ancrage d'un moyen prévu pour exercer une force sur la mandibule, lui-même fixé à un moyen de fixation présent sur un arceau.

Dans une variante de l'invention, l'appareil d'avancée mandibulaire comporte deux branches extra-orales formant chacune un arc s'étendant depuis le moyen d'ancrage à la mandibule ou depuis le lien extra-oral en direction de la partie postérieure dudit appareil, ou de la tête du patient lorsque celui-ci porte, l'appareil, lesdites branches comportant chacune au moins un moyen d'ancrage des moyens prévus pour exercer une ou plusieurs forces sur la mandibule. Dans ce mode de réalisation, les branches extra-orales suivent chacune un profil du visage du patient. Généralement, dans ce mode de réalisation, les moyens prévus pour exercer des forces sur la mandibule seront fixés aux parties des branches latérales qui encadrent la mandibule. Dans ce cas, il sera possible d'appliquer des forces latéralement à la mandibule.

Selon un mode de réalisation de l'invention, l'appareil d'avancée mandibulaire comporte au moins deux moyens d'ancrage des moyens prévus pour exercer une ou plusieurs forces prédéterminées sur la mandibule, disposés sur l'appui extra-oral sous mandibulaire dans sa partie postérieure, respectivement, de part et d'autre du plan de symétrie vertical dudit appui extra-oral sous mandibulaire.

Il est avantageux que l'appareil d'avancée mandibulaire comporte à la fois, au moins deux de ces moyens d'ancrage disposés sur l'appui extra-oral et au moins un moyen d'ancrage disposé à l'extrémité de la branche extra-orale. En effet, la position de ces points d'ancrage des moyens exerçant les forces, d'une part en avant du visage et, d'autre part, au voisinage des faces latérales de la mandibule permet d'appliquer des combinaisons de forces antéro postérieures, verticales et latérales.

Selon un mode de réalisation de l'invention, le ou les moyens de fixations du ou des moyens prévus pour exercer une ou plusieurs forces sur la mandibule et/ou le maxillaire supérieur sont disposés sur le ou les arceaux s'étendant entre les secondes branches latérales dudit appui pour la tête, c'est à dire les branches latérales destinées à être les plus éloignées du visage du patient.

Selon une caractéristique de l'invention, les moyens prévus pour exercer une ou plusieurs forces sur la mandibule sont choisis parmi des moyens élastiques tels que des élastiques d'orthodontie et/ou des moyens non élastiques tels que des tiges filetées, des tiges télescopiques, des chaînettes, des crochets munis d'anneaux d'ancrage. Les moyens élastiques étant essentiellement les moyens destinés à être ancrés sur l'appui extra-oral sous mandibulaire et les moyens non élastiques étant essentiellement les moyens destinés à être ancrés sur la branche extra-orale de l'appareil d'avancée mandibulaire. Avantageusement, les moyens prévus pour exercer des forces sur la mandibule comportent des rupteurs de forces.

Selon un mode de réalisation de l'invention, les moyens prévus pour exercer une ou plusieurs forces sur la mandibule et/ou sur le maxillaire supérieur comprennent, de plus, un moyen formant une butée contre la ou les branches extra-orales de l'appareil d'avancée mandibulaire et/ou de l'appareil d'avancée du maxillaire supérieur.

Préférentiellement, ledit moyen formant une butée est constitué par un arceau s'étendant entre lesdites premières branches latérales dudit appui pour la tête, c'est à dire les branches latérales destinées à être les plus proches du visage du patient.

La butée forme alors un pivot et permet d'obtenir un moment de force de manière à provoquer une rotation de la mandibule et/ou du maxillaire supérieur autour de l'axe du pivot lorsque les forces exercées par les moyens prévus ne sont pas horizontales. Il est donc possible d'obtenir une rotation horaire ou anti horaire de la mandibule et/ou du maxillaire supérieur.

Selon un mode de réalisation de l'invention, le moyen intra-oral d'ancrage à la mandibule et/ou le moyen intra-oral d'ancrage au maxillaire supérieur sont composés d'une gouttière, ou d'une ou plusieurs hémi-gouttières, destinées à être ancrées de manière amovible ou non sur la mandibule et/le maxillaire supérieur, de préférence sur les dents. Dans une variante de l'invention, le moyen intra-oral d'ancrage au maxillaire supérieur est composé de bracketts orthodontiques collés sur les dents.

Selon un mode de réalisation de l'invention, le dispositif comprend, de plus, un appui pour les jambes, par exemple prévu pour les maintenir fléchies, et/ou un support pour les bras ajustable de manière à ce que les bras soient positionnés au niveau ou au-dessus du coeur du patient.

Préférentiellement, l'angle α formé entre le buste du patient et la verticale au sol est compris entre 30 et 55°, préférentiellement entre 40 et 50°, plus préférentiellement entre 45 et 48°.

Préférentiellement encore, le siège, l'appui pour la tête, le support pour les bras et l'appui pour les jambes sont montés sur des axes que comporte le châssis.

Avantageusement, le châssis comporte des moyens de réglage des hauteurs respectives du siège, du support pour les bras et de l'appui pour les jambes par rapport au sol ou les uns par rapport aux autres.

L'appui pour la tête comportant les moyens de fixation des moyens exerçant les forces sur la mandibule et/ou le maxillaire, il est préférable que celui-ci soit monté de manière amovible sur le châssis du support pour le corps afin que le patient puisse l'emporter avec lui en consultation chez le praticien chargé de déterminer les forces à appliquer, c'est à dire, de déterminer la position des moyens de fixation sur l'appui pour la tête.

Préférentiellement, l'appui pour la tête est monté sur le châssis dans le prolongement du support pour la cage thoracique.

Préférentiellement encore, l'appui pour la tête comprend au moins un axe de rotation du cadre.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
La Fig. 1 représente une vue de profil d'un dispositif de traitement et/ou de soulagement des troubles respiratoires obstructifs du sommeil d'un patient selon un premier mode de réalisation de l'invention,
La Fig. 2 représente une vue de trois-quarts face d'un appui pour la tête d'un dispositif de traitement et/ou de soulagement des troubles respiratoires obstructifs du sommeil d'un patient selon un second mode de réalisation,
La Fig. 3 est une représentation schématique d'une vue de trois-quarts face d'un appui pour la tête d'un dispositif de traitement et/ou de soulagement des troubles respiratoires obstructifs du sommeil d'un patient selon le second mode de réalisation,
La Fig. 4 représente une vue de face d'un appui pour la tête d'un dispositif de traitement et/ou de soulagement des troubles respiratoires obstructifs du sommeil d'un patient selon un mode de réalisation,
La Fig. 5 représente une vue de face d'un appui pour la tête d'un dispositif de traitement et/ou de soulagement des troubles respiratoires obstructifs du sommeil d'un patient selon un autre mode de réalisation,
La Fig. 6 est une représentation schématique de profil d'un appareil d'avancée mandibulaire, d'un appareil d'avancée du maxillaire ainsi que des moyens prévus pour exercer des forces sur la mandibule et sur le maxillaire supérieur selon un mode de réalisation de l'invention,
La Fig. 7 est une représentation schématique vue de dessus d'un appareil d'avancée mandibulaire ainsi que des moyens prévus pour exercer des forces sur la mandibule selon un mode de réalisation de l'invention,
Les Figs. 8A, B, C sont des représentations schématiques vues de dessus d'appareils d'avancée mandibulaire selon différents modes de réalisation de l'invention,
La Fig. 9 est une représentation schématique vue de dessus d'un appareil d'avancée du maxillaire supérieur ainsi que des moyens prévus pour exercer des forces sur le maxillaire supérieur selon un mode de réalisation de l'invention,
Les Figs. 10A et 10B, sont des représentations schématiques vues de dessus d'appareils d'avancée du maxillaire supérieur selon différents modes de réalisation de l'invention,
La Fig. 11 est une représentation schématique des forces exercées sur le maxillaire supérieur et la mandibule dans le mode de réalisation illustré en lien avec la Fig. 6 et,
La Fig. 12 est une représentation schématique des forces exercées sur le maxillaire supérieur et la mandibule selon une variante de l'invention.

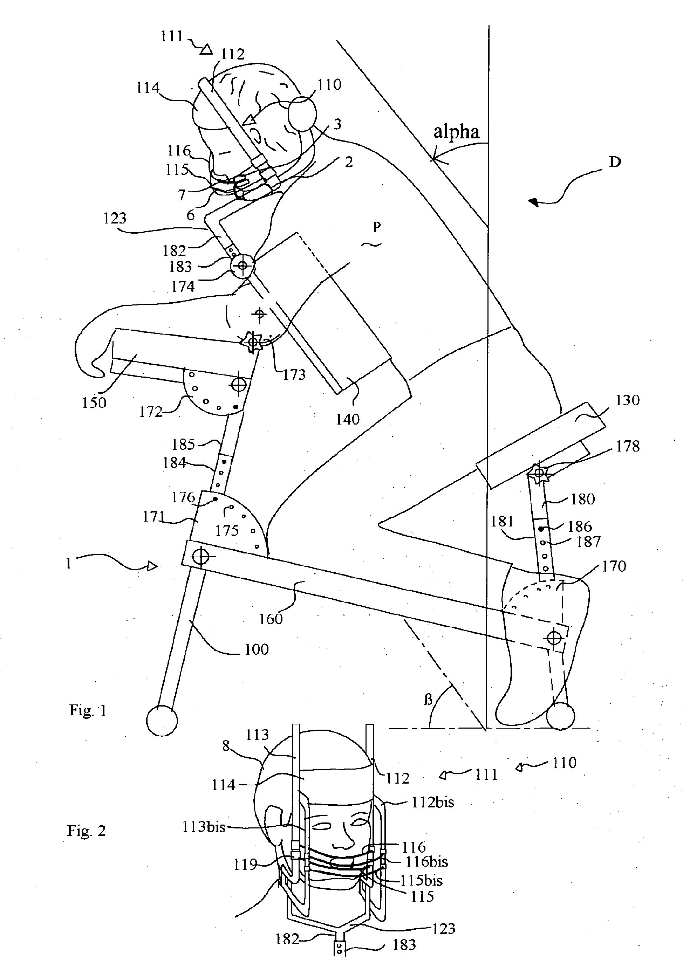

En lien avec la Fig. 1 est représenté un dispositif D de traitement et/ou de soulagement des troubles respiratoires obstructifs du sommeil. Le dispositif D est composé d'un support 1 pour le corps du patient P, d'un appareil d'avancée mandibulaire 2, d'un appareil d'avancée du maxillaire supérieur 3, lesdits appareils étant portés par le patient P.

Le support 1 est destiné à soutenir le corps du patient P dans une position proche du décubitus ventral ou une position dans laquelle les voies aériennes supérieures sont les plus ouvertes possibles.

Le patient P, installé sur le dispositif D, porte l'appareil d'avancée mandibulaire 2. Celui-ci est lié de manière amovible au support 1 par l'intermédiaire de moyens prévus pour exercer des forces sur la mandibule 6. Le patient porte encore l'appareil d'avancée du maxillaire supérieur 3 également lié de manière amovible au support 1 par l'intermédiaire de moyens prévus pour exercer des forces sur le maxillaire supérieur 7. Ainsi, les forces prévues pour engendrer un déplacement de la mandibule et du maxillaire supérieur sont exercées depuis le support 1.

Le support 1 est composé d'un châssis 100 sur lequel sont montés un appui 110 pour la tête du patient, un siège 130, un support pour la cage thoracique 140, un support pour reposer les bras 150 et un support pour reposer les jambes 160. Chacun de ces éléments est monté sur un axe de rotation que comporte le châssis 100 de manière à permettre un réglage de chacun des éléments du support 1 en fonction du gabarit du patient P et de la position dans laquelle il est souhaitable de l'installer. Ainsi, dans une position optimum le patient P, est assis sur le support 1 la tête positionnée contre l'appui pour la tête 110 et le buste est maintenu incliné vers l'avant selon un angle α, à la verticale au sol, inférieur à 90°, par exemple, selon un angle proche de 45 °.

Le châssis 100 comporte donc des moyens de réglage de la rotation du siège 130, du support pour la cage thoracique 140, du support pour reposer les jambes 160 et du support pour reposer les bras 150. Ces moyens sont composés, par exemple, d'entretoises 170, 171, 172, 173 comportant par exemple des trous, tels que le trou 175 et des moyens de blocage, tels qu'une tige 176 destinée à être insérée dans un des trous ou encore des poignées telles que la poignée 178. N'importe quel autre moyen connu de l'homme du métier permettant la rotation des éléments 130, 140, 150, 160 du support 1 et leur blocage/déblocage peut bien entendu convenir. De tels moyens sont bien connus de l'homme du métier.

L'appui pour la tête 110 est également monté sur le châssis 100 autour d'un axe de rotation à l'aide, par exemple, d'un ensemble de rotule et de disques frein 174 ou à l'aide d'une entretoise. Il est en effet important que l'appui pour la tête puisse pivoter en fonction de la physionomie du patient P.

Ces moyens de réglage de la rotation des éléments 110, 130, 140, 150 160, permettent d'ajuster l'angle de chacun des éléments par rapport au sol et/ou les angles formés entre eux de manière à adapter le dispositif au gabarit du patient P. Par exemple, l'angle ß formé entre le sol et le support pour la cage thoracique 140 permet d'obtenir l'inclinaison du buste vers l'avant selon un angle souhaité par rapport au sol.

Dans le mode de réalisation du dispositif D illustré à la Fig. 1, le siège 130, l'appui pour la tête 110, le support pour la cage thoracique 140 et le support pour reposer les bras 150 sont encore montés de manière amovible sur le châssis 100 par l'intermédiaire, par exemple, de tubes coulissants perforés 180, 181, 182, 183 , 184, 185. Des moyens de blocage sont prévus, tels que des tiges 186 prévues pour être logées dans l'un des trous 187. Ces tubes coulissants perforés et les moyens de blocage permettent d'ajuster également la hauteur de chacun des éléments par rapport au sol et/ou entre eux de manière à adapter le dispositif au gabarit du patient P.

L'appui pour la tête 110 selon un premier mode de réalisation de l'invention illustré en lien avec la Fig. 1 est composé d'un cadre 111 comportant deux branches latérales parallèles entre elles, dont la branche latérale 112 visible sur la Fig. 1. Une surface d'appui 114 au minimum pour le front du patient s'étend entre ces branches latérales. Il peut s'agir d'un tissu plus ou moins épais ou d'un coussin recevant le front ou encore d'une lanière. La surface d'appui 114 peut coulisser verticalement entre les branches latérales 112, 113 de manière à régler sa position en fonction de la taille du patient. Elle pourrait encore être mobile dans un sens vertical par rapport aux branches 112,113.

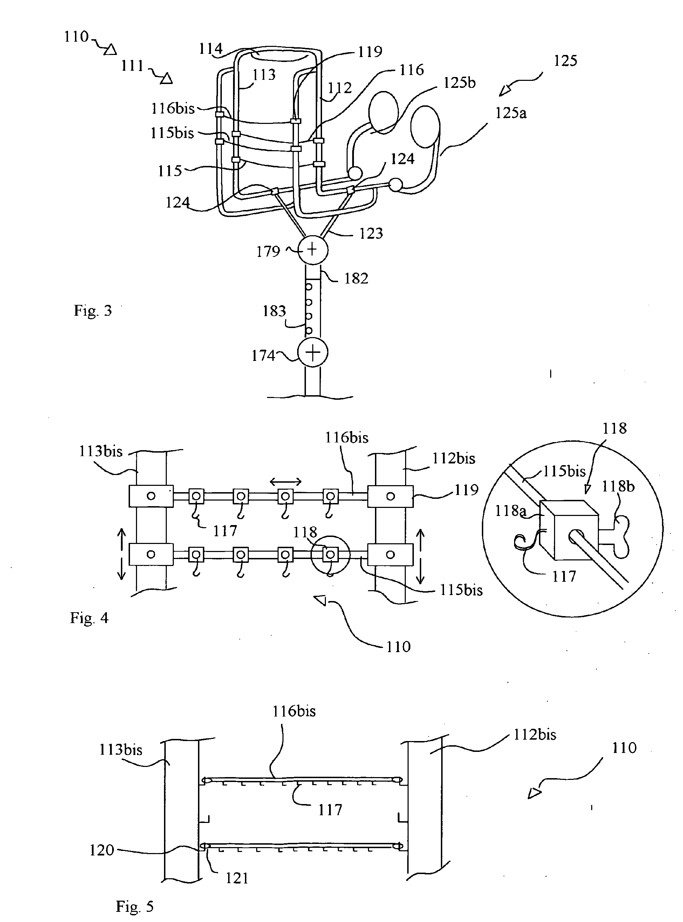

Un appui pour la tête 110 selon un second mode de réalisation de l'invention est encore illustré en lien avec les Figs. 2 et 3. Dans ce mode de réalisation, l'appui pour la tête 110 se compose d'un cadre 111 comportant deux premières branches latérales parallèles entre elles 112, 113 ainsi que deux secondes branches latérales 112 bis, 113 bis parallèles entre elles. Les branches latérales 112 bis et 113 bis sont en vis à vis avec les branches latérales 112 et 113, respectivement. Les branches 112bis, 113 bis sont ainsi dans un plan qui est parallèle au plan des branches 112, 113 et qui est postérieur au plan des branches latérales 112, 113 par rapport au visage du patient P, c'est à dire plus éloigné du visage.

Dans les modes de réalisation représentés sur les Figs. 1 et 2, les branches présentent un coude et s'étendent depuis le sommet de la tête du patient P jusqu'en avant du visage (Fig. 1) ou jusqu'en arrière (Fig. 2, Fig. 3). Dans ces configurations, une partie au moins des branches latérales 112, 113, et/ ou 112 bis, 113 bis encadre la mandibule et le maxillaire supérieur.

L'appui pour la tête 110 comprend une partie inférieure 123, avantageusement en forme de Y, prévue pour être montée de manière amovible sur le châssis 100 du support 1 dans le prolongement du support pour la cage thoracique 140, par exemple à l'aide d'un jeu de tubes coulissants perforés 182, 183 comme décrit précédemment.

Il peut être avantageux de prévoir un axe de pivotement supplémentaire du cadre 111, par exemple à l'aide d'un ensemble de rotules-disque freins 179, sur l'appui pour la tête 110 et, plus, précisément sur la partie inférieure 123, de manière à faciliter encore le positionnement de celui-ci relativement au support pour le corps 1 (Fig. 3).

Par ailleurs, le cadre 111 est monté coulissant sur la partie inférieure 123 de manière à pouvoir être déplacé dans le sens antéro-postérieur relativement à la position de la tête du patient. Des moyens autorisent le coulissement, tels que des écrous 124 à l'intérieur desquels sont enfilées les branches 112, 113. Des vis papillons vissées dans les écrous permettent le verrouillage de la position du cadre 111 sur la partie inférieure 123.

Un appui occipital 125 est en outre prévu de manière à soutenir la partie inférieure de la tête du patient. Il est composé de deux bras 125a, 125b reliés respectivement aux branches latérales 112, 113.

Sur l'appui pour la tête 110 sont également prévus des arceaux 115, 116 reliant une branche 112 à l'autre 113 et perpendiculaires à celles-ci (Fig. 1, Fig. 2, Fig. 3). De même, des arceaux 115 bis, 116 bis relient entre elles les branches 112 bis et 113 bis (Fig. 2, Fig. 3). Les arceaux font face au visage du patient P et entourent la quasi-totalité de celui-ci. Dans le mode de réalisation illustré en lien avec les Figs. 2 et 3, les arceaux 115 bis, 116 bis sont en arrière des arceaux 115, 116 par rapport au visage.

L'appui pour la tête 110 selon le second mode de réalisation est représenté plus en détail sur les Figs. 4 et 5. Les arceaux 115 bis, 116 bis qui relient les branches 112 bis, 113 bis comportent des moyens de fixation 117 destinés à fixer des moyens prévus pour exercer des forces sur la mandibule et/ou le maxillaire supérieur 6 et 7, respectivement. Ces moyens de fixation 117 sont, par exemple, des crochets. Un moyen de réglage 118 de la position des moyens de fixation 117 le long d'un arceau 115 bis ou 116 bis permet aux moyens 117 d'être déplacés le long de cet arceau. Il peut, par exemple, s'agir d'un jeu d'écrou 118a et de vis papillon 118b, l'écrou 118a supportant le moyen de fixation 117, la vis papillon 118b pouvant être vissée dans l'écrou 118a de manière à bloquer le déplacement du moyen de fixation 117.

De même, les arceaux 115 bis, 116 bis sont préférentiellement montés coulissants le long d'une partie au moins des branches latérales 112 bis, 113 bis. Des moyens de réglage 119 de leur position sur les branches 112 bis, 113 bis sont également prévus, par exemple, du type écrou/vis papillon. Ces moyens 119 peuvent également permettre le déplacement des arceaux 115 bis et 116 bis dans un sens vertical par rapport aux branches latérales 112 bis, 113 bis.

La possibilité de déplacer à la fois les moyens de fixation des moyens exerçant des forces le long des arceaux, donc horizontalement par rapport au visage du patient, et les arceaux eux-mêmes entre les branches latérales, donc verticalement par rapport au visage du patient, offre une multitude de possibilités d'orientation des forces exercées sur les deux maxillaires.

Dans une variante de l'invention représentée à la Fig. 5, les moyens de fixation 117 sont fixes mais régulièrement répartis le long des arceaux 115 bis, 116 bis. De même, les arceaux 115 bis, 116 bis peuvent être fixes le long des branches 112 bis, 113 bis. Dans ce cas, différents point d'ancrage 120 seront prévus le long des branches, tels que crochets ou tout moyen équivalent, à l'aide de boucles 121.

La fixation des arceaux 115 et 116 le long des branches latérales 112 et 113 est réalisée à l'aide de moyens identiques à ceux décrits concernant les arceaux 115 bis et 116 bis.

Le nombre d'arceaux entre les branches 112 bis, 113 bis et 112, 113 est variable, ceux-ci pouvant être ôtés ou mis en place facilement à l'aide de moyens appropriés. Généralement un arceau 115 bis monté entre les branches latérales 112 bis, 113 bis ainsi qu'un arceau 115 monté entre les branches latérales 112, 113 seront utilisés lors du traitement appliqué sur la mandibule du patient. De même, un arceau 116 bis monté entre les branches latérales 112 bis, 113 bis ainsi qu'un arceau 116 monté entre les branches latérales 112, 113 seront utilisés lors du traitement appliqué sur le maxillaire supérieur.

Des moyens de fixation 117 des moyens prévus pour exercer des forces sur la mandibule 6 et/ou le maxillaire supérieur 7 peuvent être présents également sur les parties des branches 112 bis, 113 bis qui encadrent la mandibule et/ou le maxillaire supérieur.

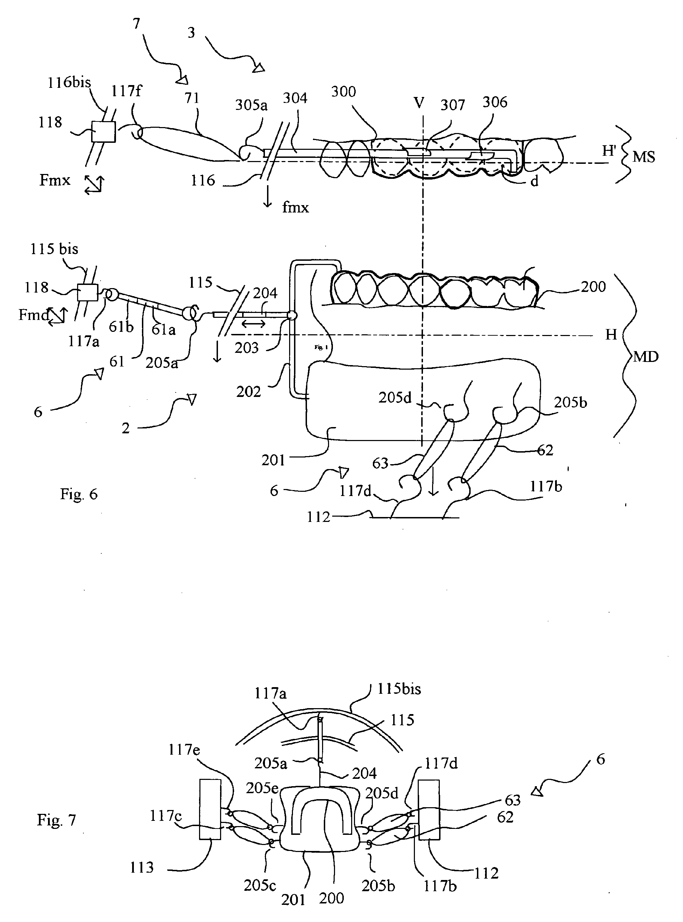

L'appareil d'avancée mandibulaire 2 du dispositif D est composé d'un moyen intra-oral 200 d'ancrage à la mandibule MD et d'un appui extra-oral sous mandibulaire 201, reliés entre eux par un lien extra-oral 202 (Fig. 6). Le moyen intra-oral 200 d'ancrage à la mandibule MD est typiquement une gouttière, ou encore deux hémi-gouttières, thermoformées aptes à être ancrées de manière non définitive sur les dents d, ou encore sur les gencives. L'appui extra-oral sous mandibulaire 201, ou mentonnière, est prévu pour recevoir le menton et/ou le rebord sous mandibulaire du patient P. Le moyen intra-oral 200 et l'appui extra-oral 201 sont reliés entre eux par un lien extra-oral rigide 202, ou semi rigide, comportant une charnière 203. La charnière 203 facilite la mise en place de l'appareil d'avancée mandibulaire 2 sur le patient. Au moins une branche extra-orale 204 se prolonge depuis le lien extra-oral 202 dans un plan horizontal à l'appareil d'avancée mandibulaire, hors de la cavité buccale du patient, en avant du visage, en direction de l'arceau 115 bis qui s'étend entre les branches 112 bis et 113 bis.

Un moyen d'ancrage 205a, tel qu'un crochet, est prévu à l'extrémité de la branche extra-orale 204 pour fixer un moyen prévu pour exercer une force sur la mandibule 61. Celui-ci est d'autre part fixé sur l'arceau 115 bis par l'intermédiaire d'un moyen de fixation 117a.

La force Fmd exercée sur la mandibule par le moyen 61 est ici une force oblique dirigée vers l'avant du visage, c'est à dire dans une direction postéro-antérieure, et vers le haut.

Les moyens prévus pour exercer une force sur la mandibule comprennent encore l'arceau 115. En effet, celui-ci vient en butée contre la branche extra-orale 204, pardessus celle-ci, et exerce alors une force verticale fmd vers le bas sur celle-ci (Fig. 6).

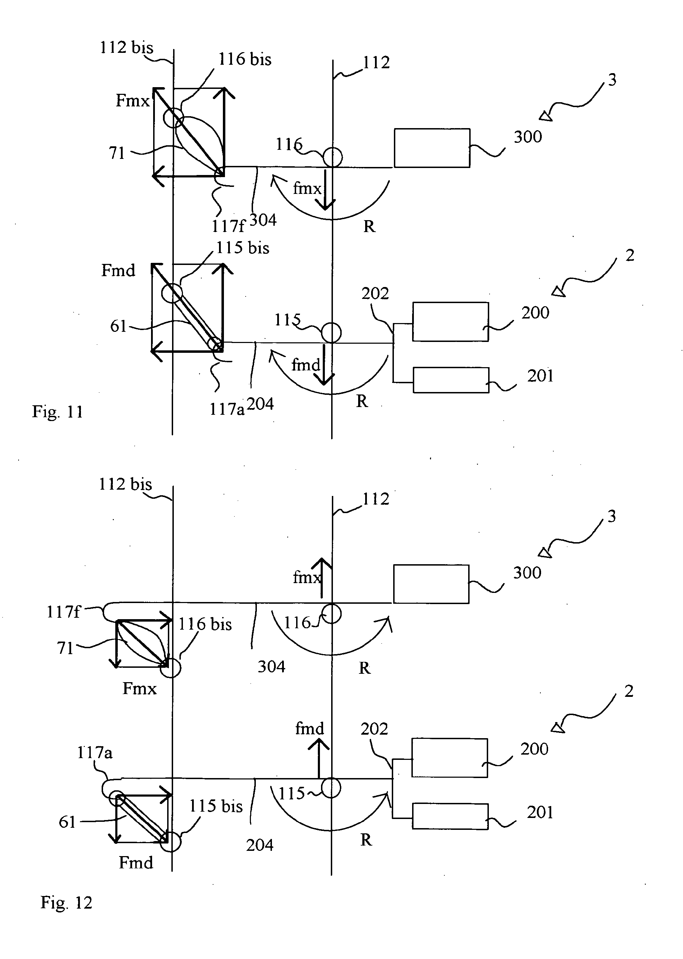

Les forces exercées sur la mandibule, dans le mode de réalisation illustré à la Fig.6 sont illustrées plus en détail sur la Fig. 11. La combinaison de la force Fmd (oblique vers l'avant et le haut) exercée par le moyen prévu pour exercer des forces 61 et de la force fmd exercée par l'arceau 115 (verticale vers le bas) permet d'obtenir une rotation horaire R de la mandibule ainsi qu'une translation vers l'avant. L'arceau 115 formant butée crée en effet un axe de rotation, comme cela est représenté sur la figure.

Le moyen prévu pour exercer la force sur la mandibule 61 par l'intermédiaire de la branche extra-orale 204 est un moyen non élastique. Il peut s'agir d'un moyen de longueur réglable tel qu'une tige filetée 61a vissée dans un tube taraudé 61b. Le moyen 61 est muni de boucles à ses extrémités pour permettre sa fixation sur le moyen d'ancrage 205 et le moyen de fixation 117 constitués par des crochets (Fig. 6). Un moyen prévu pour exercer une force sur la mandibule 6 pourrait également se présenter sous la forme d'un crochet muni à ses extrémités d'anneaux. De tels crochets sont utilisés couramment dans le domaine de l'orthodontie et se présentent sous différentes tailles. Des rupteurs de forces (non représentés) sont préférentiellement prévus.

Par ailleurs, la branche extra-orale 204 peut elle-même être réglable en longueur, par exemple, par l'intermédiaire d'un jeu de filetage/tube taraudé.

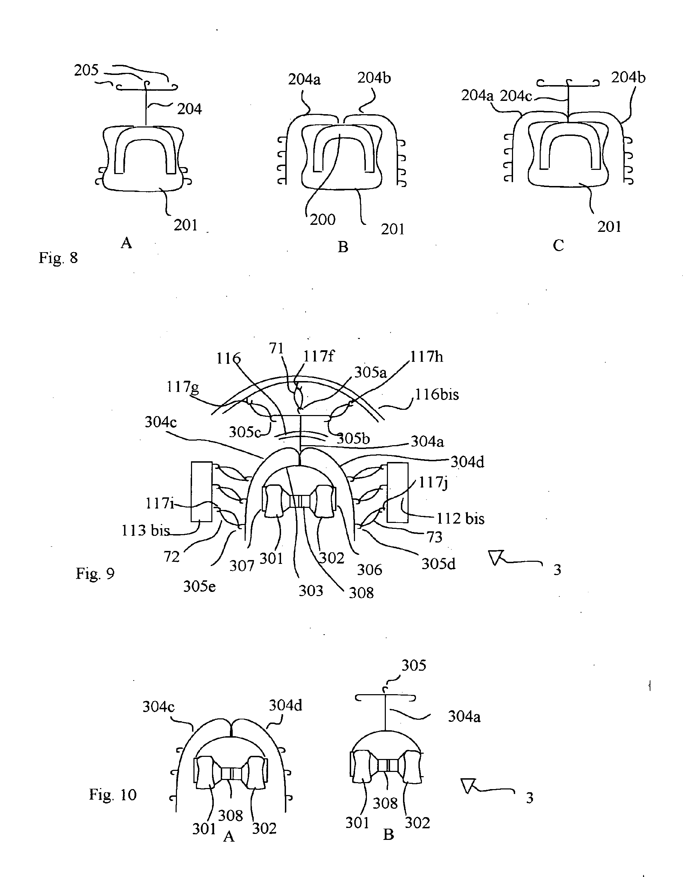

Des moyens d'ancrage 205b, 205c, 205d, 205e sont encore prévus sur l'appui extra-oral sous mandibulaire 201, par exemple, dans sa partie postérieure de part et d'autre du plan de symétrie vertical V de l'appareil 2 (Figs. 7 et 8).

Les moyens d'ancrage 205b, 205d, 205c, 205e permettent d'ancrer sur l'appui sous-mandibulaire 201 des moyens prévus pour exercer une force sur la mandibule 6, tels que les moyens 62 et 63, lesquels sont également fixés à des moyens de fixation 117b, 117d et 117c, 117e présents sur les branches latérales 112 bis, 113 bis, respectivement, de l'appui pour la tête 110. Les forces exercées par les moyens 62 et 63 sont ici des forces de traction obliques dirigées vers le bas et l'avant.

Les moyens prévus pour exercer une ou plusieurs forces sur la mandibule 62, 63 par l'intermédiaire de l'appui sous mandibulaire 201 sont des moyens élastiques. Il s'agit là encore d'élastiques couramment utilisés en orthodontie.

Il est donc possible d'appliquer des forces extra-orales depuis l'appui pour la tête 110 du support 1 à la fois sur les faces latérales de la mandibule et dans le plan médian de la mandibule.

La force Fmd exercée sur la mandibule par le moyen 61 peut être alternativement une force dirigée vers l'arrière (sens antéro-postérieur) et vers le bas (Fig. 12). La force fmd exercée par l'arceau 115 est dans ce cas une force verticale vers le haut, celui-ci étant appuyé sur la branche extra-orale 204 via le dessous de celle-ci. On obtient alors une rotation antihoraire R et une translation dans le sens antéro-postérieur de la mandibule.

Selon la position de l'arceau 115 bis le long des branches 112 bis, 113 bis, selon la position du moyen de fixation 117a le long de l'arceau 115 bis, et selon la position de l'arceau 115 entre les branches latérales 112, 113, il est possible de faire varier les composantes des forces exercées, en fonction du traitement à appliquer au patient.

Dans des variantes de l'invention représentées sur les Fig. 8A à 8C, l'appareil d'avancée mandibulaire 2 comprend une branche extra-orale 204 dont l'extrémité forme une croix (Fig. 8A). Dans ce cas, il est possible de prévoir plusieurs moyens d'ancrage 205. Il peut encore comporter deux branches extra-orales 204a, 204b formant chacune un arc s'étendant depuis le moyen d'ancrage à la mandibule 200 (Fig. 8B), ou depuis le lien extra-oral 202 (Fig. 8C), en direction de la partie postérieure de l'appareil 2, les branches extra-orales 204a, 204b, 204c comportant chacune au moins un moyen d'ancrage 205 des moyens prévus pour exercer une ou plusieurs forces sur la mandibule 61.

L'appareil d'avancée du maxillaire 3 du dispositif D est composé d'un moyen intra-oral 300 d'ancrage au maxillaire supérieur MS, formé par exemple par deux hémi-gouttières thermoformées 301, 302 aptes à être ancrées sur les dents d, ou sur les gencives, et reliées entre elles par un arc intra-oral 303 (Fig. 6 ; Fig. 9 ; Fig. 10) ou par un vérin 308. Au moins une branche extra-orale 304 se prolonge depuis l'arc intra-oral 303, dans le plan horizontal H' de l'appareil 3, en avant du visage, hors de la cavité buccale du patient, dont seules les dents sont représentées sur la Fig. 6 afin de ne pas nuire à la clarté de la figure, en direction par exemple d'un arceau 116 bis.

La branche extra-orale 304 est fixée de manière amovible au moyen intra-oral 300, par exemple, par l'intermédiaire de l'arc 303 (voir Figs. 9 et 10). Le moyen intra-oral 300 est préférentiellement collé sur les dents. Par exemple, des gouttières 306, 307 sont fixées sur les hémi-gouttières 301, 302 dans lesquelles les extrémités de l'arc intra oral 303 peuvent être glissées (Fig. 6).

Des moyens d'ancrage, tels que le moyen 305a, des moyens prévus pour exercer une ou plusieurs forces sur le maxillaire supérieur 7 sont prévus à l'extrémité de la branche extra-orale 304 (Fig.).

Les moyens prévus pour exercer une ou plusieurs forces sur le maxillaire supérieur 7, tels que le moyen 71, incluent des moyens élastiques, préférentiellement pourvus de rupteurs de forces (non représentés). De tels élastiques sont couramment utilisés en orthodontie. Le moyen prévu pour exercer une force sur la maxillaire 71 est fixé d'une part, au moyen d'ancrage 305a à l'extrémité de la branche extra-orale 304 et d'autre part, au moyen de fixation 117f de l'arceau 116 bis.

La force Fmx exercée sur le maxillaire supérieur par le moyen 71 est ici une force oblique dirigée vers l'avant du visage, c'est à dire postéro-antérieure, et vers le haut.

Les moyens 7 prévus pour exercer une force sur le maxillaire supérieur comprennent encore l'arceau 116. En effet, celui-ci forme une butée contre la branche extra-orale 304 et exerce une force verticale fmx vers le bas sur celle-ci (Fig. 6).

Les forces exercées sur le maxillaire supérieur, dans le mode de réalisation illustré à la Fig. 6, sont illustrées plus en détail sur la Fig. 11. La combinaison de la force Fmx exercée par le moyen prévu pour exercer des forces 71 (oblique vers le haut et l'avant) et de la force fmx exercée par l'arceau 116 (verticale vers le bas) permet d'obtenir une rotation horaire R du maxillaire supérieur ainsi qu'une translation vers l'avant. L'arceau 116 formant butée crée en effet un axe de rotation, comme cela est représenté sur la figure 11.

La force Fmx exercée sur le maxillaire supérieur MS par le moyen 71 peut être alternativement une force dirigée vers l'arrière (sens antéro-postérieur) et vers le bas. L'arceau 116 dans ce cas est en appui sur la branche extra-orale 304 via le dessous de celle-ci et exerce une force fmx verticale vers le haut (Fig. 12). On obtient alors une rotation antihoraire et une translation dans le sens antéro-postérieur.

Là encore, selon la position de l'arceau 116 bis le long des branches 112 bis, 113 bis, selon la position du moyen de fixation 117f le long de l'arceau 116 bis, et selon la position de l'arceau 116 entre les branches 112, 113 il est possible de faire varier les composantes des forces exercées.

En outre, l'utilisation d'un vérin 308 entre les gouttières ou hémi-gouttières 301, 302, permet d'obtenir conjointement à l'avancée du maxillaire supérieur, une expansion du palais. Tout autre moyen qu'un vérin connu pour provoquer une expansion palatine peut bien entendu être utilisé.

Dans des variantes de l'invention représentées sur les Figs. 9 et 10, l'appareil d'avancée du maxillaire supérieur 3 comprend une branche extra-orale 304 dont l'extrémité forme une croix (Figs. 9 et 10B) et/ou deux branches extra-orales 304b, 304c lesquelles forment chacune un arc s'étendant depuis le moyen intra-oral 300 en direction de la partie postérieure de l'appareil 3 (Fig. 10A). Les branches extra-orales 304a, 304b, 304c comportent chacune au moins un moyen d'ancrage 305a, 305b, 305c des moyens prévus pour exercer une ou plusieurs forces sur le maxillaire supérieur, tels qu'un moyen élastique 71.

Lorsque l'appareil d'avancée du maxillaire supérieur 3 comprend deux branches extra-orales 304a, 304b formant chacune un arc s'étendant en direction de la partie postérieure, les moyens prévus pour exercer des forces sur le maxillaire supérieur, tels que les moyens 72, 73, sont fixés d'une part, aux moyens d'ancrage 305 (tels que les moyens 305e et 305d) des branches 304b, 304c et, d'autre part, aux moyens de fixation 117 (tels que les moyens 117i et 117j) des branches latérales 112 bis, 113 bis. Dans ce cas encore, étant donné que les positions des moyens de fixation 117 sont variables et réglables le long des arceaux 115, 116 et/ou des branches latérales 112, 113 et que les arceaux 115, 116 peuvent être fixés à différents endroits sur les branches 112, 113, il est possible de déterminer avec précision les directions des forces à exercer sur le maxillaire supérieur.

Les moyens de fixation 117 ainsi que les moyens d'ancrage 205 permettant de fixer les moyens prévus pour exercer des forces sur la mandibule 6 peuvent encore, au lieu de crochets, se présenter sous la forme d'écrous. Dans ce cas, les moyens prévus pour exercer des forces sur la mandibule 6 présentent à leurs extrémités des tiges filetées.

Les réglages des positions des moyens de fixation 117 sur les arceaux 115 bis, 116 bis, des positions des arceaux 115, 116, 115 bis, 116 bis, relativement aux branches 112, 113, 112 bis, 113 bis ainsi que le choix des moyens prévus pour exercer les forces sur la mandibule et/ou le maxillaire supérieur 6, 7 sont réalisables chez un praticien aisément. En effet, ceux-ci étant prévus pour être fixés à l'appui pour la tête 110 lequel est amovible du support 1, il suffit au patient d'emporter l'appui pour la tête 110 en consultation. Le praticien détermine en fonction du patient les combinaisons de forces à appliquer.

## Revendications

1. Dispositif (D) de traitement et/ou de soulagement des troubles respiratoires obstructifs du sommeil d'un patient (P) **caractérisé en ce qu'**il comprend :
- un support (1) pour le corps d'un patient dont le châssis (100) comprend un appui pour la tête (110) du patient, un siège (130) et un support pour la cage thoracique (140) lesquels sont montés sur le châssis (100) de manière à ce que lorsque le patient prend place et s'assoit sur ledit support (1) et positionne sa tête contre l'appui pour la tête (110), son buste soit maintenu incliné vers l'avant selon un angle α, à la verticale au sol, inférieur à 90°,
- un appareil d'avancée mandibulaire (2) composé d'un moyen extra-oral d'ancrage à la mandibule et/ou d'un moyen intra-oral d'ancrage à la mandibule (200), et/ou
- un appareil d'avancée du maxillaire supérieur (3) composé d'un moyen intra-oral d'ancrage au maxillaire supérieur (300),
- des moyens de fixation (117), sur ledit appui pour la tête (110), d'un ou plusieurs moyens prévus pour exercer une ou plusieurs forces prédéterminées sur la mandibule (6) et/ou le maxillaire supérieur (7) par l'intermédiaire de l'appareil d'avancée mandibulaire (2) et/ou de l'appareil d'avancée du maxillaire supérieur (3).

2. Dispositif (D) selon la revendication précédente, **caractérisé en ce que** le moyen extra-oral d'ancrage à la mandibule est composé d'au moins un implant transcutané.

3. Dispositif (D) selon la revendication 1, **caractérisé en ce que** l'appareil d'avancée mandibulaire (2) est composé d'un moyen intra-oral d'ancrage à la mandibule (200) et d'un appui extra-oral sous mandibulaire (201), reliés entre eux par un lien extra-oral (202) comportant préférentiellement une charnière (203).

4. Dispositif (D) selon l'une des revendications précédentes, **caractérisé en ce que** l'appui pour la tête (110) est composé d'un cadre (111) comportant deux premières branches latérales (112, 113), parallèles entre elles, et une surface d'appui (114) pour le front du patient s'étendant entre lesdites premières branches latérales (112, 113), une partie au moins des branches latérales (112, 113) encadrant la mandibule (MD) et/ou le maxillaire supérieur (MS) du patient lorsque celui-ci prend place sur ledit support et positionne son front contre la surface d'appui.

5. Dispositif (D) selon la revendication 4, **caractérisé en ce que** lesdites branches latérales (112, 113) dudit cadre (111) sont doublées, respectivement, de deux secondes branches latérales parallèles entre elles (112bis, 113bis), ces dernières se trouvant dans un plan parallèle au plan contenant les premières branches latérales (112,113).

6. Dispositif (D) selon l'une des revendications 4 à 5, **caractérisé en ce qu'**au moins un arceau (115, 116) relie lesdites premières branches latérales (112, 113) entre elles et/ou au moins un arceau (115bis, 116bis) relie lesdites secondes branches latérales (112bis, 113bis) entre elles, de manière à ce que ledit ou lesdits arceaux (115, 116, 115bis, 116bis) se trouvent face au visage dudit patient (P) lorsque celui-ci prend place sur ledit support (1) et positionne son front contre la surface d'appui (114), lesdits arceaux (115, 116, 115bis, 116bis) étant montés mobiles le long des branches latérales (112, 113, 112bis, 113bis).

7. Dispositif (D) selon la revendication 6, **caractérisé en ce que** lesdits moyens de fixation des moyens prévus pour exercer une ou plusieurs forces prédéterminées (117) sont disposés sur au moins un desdits arceaux (115, 116, 115bis, 116bis) et sont préférentiellement mobiles le long desdits arceaux.

8. Dispositif selon l'une des revendications 4 à 7, **caractérisé en ce que** lesdites premières et/ou secondes branches latérales (112bis, 113 bis) de l'appui pour la tête (110) comportent chacune au moins un moyen de fixation (117) des moyens prévus pour exercer une ou plusieurs forces sur la mandibule (6).

9. Dispositif (D) selon l'une des revendications précédentes, **caractérisé en ce que** l'appareil d'avancée du maxillaire supérieur (3) comprend au moins une branche extra-orale 304), préférentiellement amovible, se projetant depuis le moyen intra-oral d'ancrage au maxillaire supérieur (300) en avant du visage du patient lorsque celui-ci porte ledit appareil (3), à l'extrémité de laquelle sont prévus un ou plusieurs moyens d'ancrage (305a) des moyens prévus pour exercer une ou plusieurs forces prédéterminées sur le maxillaire supérieur (7).

10. Dispositif (D) selon l'une des revendications précédentes, **caractérisé en ce que** les moyens prévus pour exercer une ou plusieurs forces prédéterminées sur le maxillaire supérieur (7) sont des élastiques orthodontiques.

11. Dispositif (D) selon l'une des revendications 3 à 10, **caractérisé en ce que** l'appareil d'avancée mandibulaire (2) comporte au moins une branche extra-orale (204), se projetant depuis le moyen intra-oral d'ancrage à la mandibule (200), ou depuis le lien extra-oral (202), en avant du visage du patient lorsque celui-ci porte ledit appareil (2), à l'extrémité de laquelle sont prévus un ou plusieurs moyens d'ancrage (205) des moyens prévus pour exercer une ou plusieurs forces prédéterminées sur la mandibule (6).

12. Dispositif (D) selon l'une des revendications 2 à 11, **caractérisé en ce que** l'appareil d'avancée mandibulaire (2) comporte au moins deux moyens d'ancrage (205d, 205b) des moyens prévus pour exercer une ou plusieurs forces prédéterminées sur la mandibule (6), disposés sur l'appui extra-oral sous mandibulaire (201), préférentiellement dans sa partie postérieure de part et d'autre, respectivement, du plan de symétrie vertical (V) dudit appui extra-oral sous mandibulaire (201), ou disposés sur ledit moyen extra-oral d'ancrage à la mandibule.

13. Dispositif selon l'une des revendications 7 à 12, **caractérisé en ce que** le ou les moyens de fixations (117) du ou des moyens prévus pour exercer une ou plusieurs forces sur la mandibule (6) et ou le maxillaire (7) sont disposés sur le ou les arceaux (115bis, 116bis) qui s'étendent entre lesdites secondes branches latérales (112bis, 113bis) dudit appui pour la tête (110).

14. Dispositif (D) selon l'une des revendications précédentes, **caractérisé en ce que** les moyens prévus pour exercer une ou plusieurs forces sur la mandibule (6) comprennent des moyens élastiques tels que des élastiques d'orthodontie et/ou des moyens non élastiques tels que des tiges filetées, des tiges télescopiques, des chaînettes, des crochets munis d'anneaux d'ancrage.

15. Dispositif (D) selon l'une des revendications précédentes, **caractérisé en ce que** les moyens prévus pour exercer une ou plusieurs forces sur la mandibule (6) et/ou sur le maxillaire supérieur (7) comprennent, de plus, un moyen formant une butée contre la ou les branches extra-orales (204, 304) de l'appareil d'avancée mandibulaire (2) et/ou de l'appareil d'avancée du maxillaire supérieur (3) qui est constitué d'un arceau (115) s'étendant entre lesdites premières branches latérales (112, 113) dudit appui pour la tête (110).

16. Dispositif (D) selon l'une des revendications précédentes, **caractérisé en ce que** le moyen intra-oral d'ancrage à la mandibule (200) et/ou le moyen intra-oral d'ancrage au maxillaire supérieur (300) est composé d'une gouttière, ou hémi-gouttière, destinée à être ancrée de manière amovible ou non sur la mandibule (MD) et/le maxillaire supérieur (MS), de préférence sur les dents (d).

## Patentansprüche

1. Vorrichtung (D) zur Behandlung und/oder Linderung von obstruktiven respiratorischen Schlafstörungen eines Patienten (P), **dadurch gekennzeichnet, dass** sie aufweist:
- eine Stütze (1) für den Körper eines Patienten, deren Gestell (100) eine Auflage für den Kopf (110) des Patienten, einen Sitz (130) und eine Stütze für den Brustkorb (140) aufweist, die so an dem Gestell (100) montiert sind, dass, wenn der Patient Platz nimmt und sich auf die Stütze (1) setzt und seinen Kopf gegen die Auflage für den Kopf (110) lehnt, sein Oberkörper in einem Winkel α, der zu der Senkrechten zum Boden kleiner als 90° ist, nach vorne geneigt gehalten wird,
- eine Unterkiefer-Protrusionseinrichtung (2), die aus einem extra-oralen Mittel zur Verankerung am Unterkiefer und/oder einem intra-oralen Mittel zur Verankerung am Unterkiefer (200) besteht,
und/oder
- eine Oberkiefer-Protrusionseinrichtung (3), die aus einem intra-oralen Mittel zur Verankerung am Oberkiefer (300) besteht,
- Befestigungsmittel (117) an der Auflage für den Kopf (110) aus einem oder mehreren Mitteln, die zum Ausüben einer oder mehrerer vordefinierter Kräfte auf den Unterkiefer (6) und/oder den Oberkiefer (7) mittels der Unterkiefer-Protrusionseinrichtung (2) und/oder der Oberkiefer-Protrusionseinrichtung (3) vorgesehen sind.

2. Vorrichtung (D) nach dem vorhergehenden Anspruch, **dadurch gekennzeichnet, dass** das extra-orale Mittel zur Verankerung am Unterkiefer aus mindestens einem transkutanen Implantat besteht.

3. Vorrichtung (D) nach Anspruch 1, **dadurch gekennzeichnet, dass** die Unterkiefer-Protrusionseinrichtung (2) aus einem intra-oralen Mittel zur Verankerung am Unterkiefer (200) und einer extra-oralen Auflage unter dem Unterkiefer (201) besteht, die über eine extra-orale Verbindung (202) miteinander verbunden sind, die vorzugsweise ein Scharnier (203) aufweist.

4. Vorrichtung (D) nach einem der vorhergehenden Ansprüche, **dadurch gekennzeichnet, dass** die Auflage für den Kopf (110) aus einem Rahmen (111) besteht, der zwei zueinander parallele erste seitliche Verzweigungen (112, 113) und eine Auflagefläche (114) für die Stirn des Patienten aufweist, die sich zwischen den ersten seitlichen Verzweigungen (112, 113) erstreckt, wobei mindestens ein Teil der seitlichen Verzweigungen (112, 113) den Unterkiefer (MD) und/oder den Oberkiefer (MS) des Patienten einrahmt, wenn dieser auf der Stütze Platz nimmt und seine Stirn gegen die Auflagefläche lehnt.

5. Vorrichtung (D) nach Anspruch 4, **dadurch gekennzeichnet, dass** die seitlichen Verzweigungen (112, 113) des Rahmens (111) jeweils durch zwei zueinander parallele zweite seitliche Verzweigungen (112bis, 113bis) verdoppelt werden, wobei sich Letztere in einer Ebene befinden, die parallel zu der Ebene verläuft, die die ersten seitlichen Verzweigungen (112, 113) enthält.

6. Vorrichtung (D) nach einem der Ansprüche 4 bis 5, **dadurch gekennzeichnet, dass** mindestens ein Bügel (115, 116) die ersten seitlichen Verzweigungen (112, 113) miteinander verbindet und/oder mindestens ein Bügel (115bis, 116bis) die zweiten seitlichen Verzweigungen (112bis, 113bis) miteinander verbindet, sodass der oder die Bügel (115, 116, 115bis, 116bis) sich gegenüber dem Gesicht des Patienten (P) befinden, wenn dieser auf der Stütze (1) Platz nimmt und seine Stirn gegen die Auflagefläche (114) lehnt, wobei die Bügel (115, 116, 115bis, 116bis) entlang der seitlichen Verzweigungen (112, 113, 112bis, 113bis) beweglich montiert sind.

7. Vorrichtung (D) nach Anspruch 6, **dadurch gekennzeichnet, dass** die Befestigungsmittel für die Mittel, die zum Ausüben von einer oder mehreren vordefinieren Kräften (117) vorgesehen sind, auf mindestens einem der Bügel (115, 116, 115bis, 116bis) angeordnet sind und vorzugsweise entlang der Bügel beweglich sind.

8. Vorrichtung nach einem der Ansprüche 4 bis 7, **dadurch gekennzeichnet, dass** die ersten und/oder zweiten seitlichen Verzweigungen (112bis, 113bis) der Auflage für den Kopf (110) jeweils mindestens ein Befestigungsmittel (117) für die Mittel aufweisen, die zum Ausüben von einer oder mehreren Kräften auf den Unterkiefer (6) vorgesehen sind.

9. Vorrichtung (D) nach einem der vorhergehenden Ansprüche, **dadurch gekennzeichnet, dass** die Oberkiefer-Protrusionseinrichtung (3) mindestens eine extra-orale Verzweigung (304) aufweist, die vorzugsweise abnehmbar ist, die von dem intra-oralen Mittel zur Verankerung an dem Oberkiefer (300) vor das Gesicht des Patienten vorragt, wenn dieser die Vorrichtung (3) trägt, wobei an deren Ende ein oder mehrere Mittel zur Verankerung (305a) der Mittel vorgesehen sind, die zum Ausüben einer oder mehrerer vordefinierter Kräfte auf den Oberkiefer (7) vorgesehen sind.

10. Vorrichtung (D) nach einem der vorhergehenden Ansprüche, **dadurch gekennzeichnet, dass** die Mittel, die zum Ausüben einer oder mehrerer vordefinierter Kräfte auf den Oberkiefer (7) vorgesehen sind, kieferorthopädische Gummibänder sind.

11. Vorrichtung (D) nach einem der Ansprüche 3 bis 10, **dadurch gekennzeichnet, dass** die Unterkiefer-Protrusionseinrichtung (2) mindestens eine extra-orale Verzweigung (204) aufweist, die vom intra-oralen Mittel zur Verankerung am Unterkiefer (200) oder von der extra-oralen Verbindung (202) vor das Gesicht des Patienten vorragt, wenn dieser die Vorrichtung (2) trägt, wobei an deren Ende ein oder mehrere Mittel zur Verankerung (205) der Mittel vorgesehen sind, die zum Ausüben einer oder mehrerer vordefinierter Kräfte auf den Oberkiefer (6) vorgesehen sind

12. Vorrichtung (D) nach einem der Ansprüche 2 bis 11, **dadurch gekennzeichnet, dass** die Unterkiefer-Protrusionseinrichtung (2) mindestens zwei Mittel zur Verankerung (205d, 205b) der Mittel aufweist, die zum Ausüben einer oder mehrerer vordefinierter Kräfte auf den Unterkiefer (6) vorgesehen sind, die auf der extra-oralen Auflage unter dem Unterkiefer (201) angeordnet sind, vorzugsweise jeweils in deren rückwärtigem Teil einerseits und andererseits auf der vertikalen Symmetrieebene (V) der extra-oralen Auflage unter dem Unterkiefer (201) oder auf dem extra-oralen Mittel zur Verankerung am Unterkiefer angeordnet sind.

13. Vorrichtung nach einem der Ansprüche 7 bis 12, **dadurch gekennzeichnet, dass** das oder die Befestigungsmittel (117) des oder der Mittel(s), die zum Ausüben einer oder mehrerer Kräfte auf den Unterkiefer (6) und/oder den Oberkiefer (7) vorgesehen sind, auf dem oder den Bögen (115bis, 116 bis) angeordnet sind, die sich zwischen den zweiten seitlichen Verzweigungen (112bis, 113bis) der Auflage für den Kopf (110) erstrecken.

14. Vorrichtung (D) nach einem der vorhergehenden Ansprüche, **dadurch gekennzeichnet, dass** die Mittel, die zum Ausüben einer oder mehrerer Kräfte auf den Unterkiefer (6) vorgesehen sind, elastische Mittel aufweisen, wie beispielsweise kieferorthopädische Gummibänder, und/oder nicht-elastische Mittel, wie beispielsweise Gewindestäbe, Teleskopstäbe, Kettchen, mit Verankerungsringen ausgestattete Haken.

15. Vorrichtung (D) nach einem der vorhergehenden Ansprüche, **dadurch gekennzeichnet, dass** die Mittel, die zum Ausüben einer oder mehrerer Kräfte auf den Unterkiefer (6) und/oder auf den Oberkiefer (7) vorgesehen sind, des Weiteren ein Mittel aufweisen, das einen Anschlag für die extra-orale(n) Verzweigung(en) (204, 304) der Unterkiefer-Protrusionseinrichtung (2) und/oder der Oberkiefer-Protrusionseinrichtung (3) bildet, der aus einem Bogen (115) gebildet ist, der sich zwischen den ersten seitlichen Verzweigungen (112, 113) der Auflage für den Kopf (110) erstreckt.

16. Vorrichtung (D) nach einem der vorhergehenden Ansprüche, **dadurch gekennzeichnet, dass** das intra-orale Mittel zur Verankerung am Unterkiefer (200) und/oder das intra-orale Mittel zur Verankerung am Oberkiefer (300) aus einer Rinne oder Halbrinne besteht, die dazu bestimmt ist, abnehmbar oder nichtabnehmbar auf dem Unterkiefer (MD) und/oder dem Oberkiefer (MS) vorzugsweise an den Zähnen (d) verankert zu werden.

## Claims

1. Device (D) for the treatment and/or alleviation of obstructive respiratory sleep disorders in a patient (P), **characterised in that** the device comprises:
- a support (1) for the patient's body of which the chassis (100) comprises a rest (110) for the patient's head, a seat (130) and a support (140) for the rib cage which are mounted on the chassis (100) such that when the patient sits on said support (1) and positions his/her head against the headrest (110), the patient's chest is inclined forwards at an angle α of less than 90° relative to the vertical to the ground,
- a mandible advancing device (2) formed by an extra-oral means for anchoring to the mandible and/or an intra-oral means for anchoring to the mandible (200),
and/or
- a maxilla advancing device (3) formed by intra-oral means for anchoring to the maxilla (300),
- securing means (117) for securing one or more elements to said headrest (110), said elements being provided to exert one or more pre-determined forces on the mandible (6) and/or the maxilla (7) by means of the mandible advancing device (2) and/or the maxilla advancing device (3).

2. Device (D) according to claim 1, **characterised in that** the extra-oral means for anchoring to the mandible is formed by at least one transcutaneous implant.

3. Device (D) according to claim 1, **characterised in that** the mandible advancing device (2) is formed by an intra-oral means for anchoring to the mandible (200) and an extra-oral sub-mandibular support (201), connected together by an extra-oral link (202) preferably including a hinge (203).

4. Device (D) according to any one of the preceding claims, **characterised in that** the headrest (110) is formed by a frame (111) including two first mutually-parallel side branches (112, 113), and a resting surface (114) for the patient's forehead extending between said first side branches (112, 113), at least a part of the side branches (112, 113) framing the patient's mandible (MD) and/or the maxilla (MS) when he/she sits on said support and positions his/her forehead against the resting surface.

5. Device (D) according to claim 4, **characterised in that** said side branches (112, 113) of said frame (111) are complemented, respectively, by two second mutually-parallel side branches (112bis, 113bis), the latter being situated in a plane parallel to the plane containing the first side branches (112, 113).

6. Device (D) according to any one of claims 4 to 5, **characterised in that** at least one hoop (115, 116) connects said first side branches (112, 113) together and/or at least one hoop (115bis, 116bis) connects said second side branches (112bis, 113bis) together, so that said hoop or hoops (115, 116, 115bis, 116bis) are situated in front of the face of said patient (P) when he/she sits on said support (1) and positions his/her forehead against the resting surface (114), said hoops (115, 116, 115bis, 116bis) being movably mounted along the side branches (112, 113, 112bis, 113bis).

7. Device (D) according to claim 6, **characterised in that** said means for securing the means provided to exert one or more pre-determined forces (117) are disposed on at least one of said hoops (115, 116, 115bis, 116bis) and are preferably mobile along said hoops.

8. Device according to any one of claims 4 to 7, **characterised in that** said first and/or second side branches (112bis, 113 bis) of the headrest (110) each include at least one means (117) for securing the means provided to exert one or more forces on the mandible (6).

9. Device (D) according to one of the preceding claims, **characterised in that** the maxilla advancing device (3) comprises at least one extra-oral branch (304), preferably detachable, projecting from the intra-oral means for anchoring to the maxilla (300) in front of the patient's face when he/she is wearing said device (3), at the end of which are provided one or more means for anchoring (305a) the means provided to exert one or more pre-determined forces on the maxilla (7).

10. Device (D) according to any one of the preceding claims, **characterised in that** the means provided to exert one or more pre-determined forces on the maxilla (7) are orthodontic elastics.

11. Device (D) according to any one of claims 3 to 10, **characterised in that** the mandible advancing device (2) comprises at least one extra-oral branch (204), projecting from the intra-oral means for anchoring to the mandible (200), or from the extra-oral link (202) in front of the patient's face when he/she is wearing said device (2), at the end of which are provided one or more means for anchoring (205) the means provided to exert one or more pre-determined forces on the mandible (6).

12. Device (D) according to any one of claims 2 to 11, **characterised in that** the mandible advancing device (2) includes at least two means for anchoring (205d, 205b) the means provided to exert one or more pre-determined forces on the mandible (6), disposed on the extra-oral sub-mandibular support (201), preferably in its posterior part on each side, respectively, of the vertical plane of symmetry (V) of said extra-oral sub-mandibular support (201), or disposed on said extra-oral means for anchoring to the mandible.

13. Device according to any one of claims 7 to 12, **characterised in that** the means for securing (117) the means provided to exert one or more forces on the mandible (6) and/or the maxilla (7) are disposed on the hoop or hoops (115bis, 116bis) which extend between said second side branches (112bis, 113bis) of said headrest (110).

14. Device (D) according to any one of the preceding claims, **characterised in that** the means provided to exert one or more forces on the mandible (6) comprise resilient means such as orthodontic elastics and/or non-resilient means such as threaded rods, telescopic rods, chains or hooks provided with anchoring rings.

15. Device (D) according to any one of the preceding claims, **characterised in that** the means provided to exert one or more forces on the mandible (6) and/or on the maxilla (7) additionally comprise a means forming a stop against the extra-oral branch(es) (204, 304) of the mandible advancing device (2) and/or of the maxilla advancing device (3) which is formed by a hoop (115) extending between said first side branches (112, 113) of said headrest (110).

16. Device (D) according to any one of the preceding claims, **characterised in that** the intra-oral means for anchoring to the mandible (200) and/or the intra-oral means for anchoring to the maxilla (300) are formed by a gutter, or semi-gutter, designed to be anchored in a detachable manner or otherwise on the mandible (MD) and/the maxilla (MS), preferably on the teeth (d).
